(19) Europäisches Patentamt
European Patent Office
Office européen des brevets

(11) Veröffentlichungsnummer : **0 142 667 B1**

(12) # EUROPÄISCHE PATENTSCHRIFT

(45) Veröffentlichungstag der Patentschrift : **01.02.89**

(51) Int. Cl.$^4$ : **C 07 C127/22**, A 01 N 47/34

(21) Anmeldenummer : **84111168.5**

(22) Anmeldetag : **19.09.84**

(54) **N-Benzoyl-N'-alkoxyphenylharnstoffe und ihre Verwendung zur Bekämpfung von Insekten und Akariden.**

(30) Priorität : **22.09.83 DE 3334224**
**18.02.84 DE 3405878**

(43) Veröffentlichungstag der Anmeldung : **29.05.85 Patentblatt 85/22**

(45) Bekanntmachung des Hinweises auf die Patenterteilung : **01.02.89 Patentblatt 89/05**

(84) Benannte Vertragsstaaten : **BE CH DE FR GB IT LI NL**

(56) Entgegenhaltungen :
**US-A- 4 399 152**
**JOURNAL OF AGRICULTURAL AND FOOD CHEMISTRY, Band 21, Nr. 3, Mai/Juni 1973, Seiten 348-354, California, US; K. WELLINGA et al.: "Synthesis and laboratory evaluation of 1-(2,6-disubsituted benzoyl)-3-phenylureas, a new class of insecticides. I. 1-(2,6-dichlorobenzoyl)-3-phenylureas"**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Lange, Arno, Dr.**
**Oberes Geistal 3 b**
**D-6702 Bad Duerkheim (DE)**
Erfinder : **Will, Wolfgang, Dr.**
**Schulstrasse 46**
**D-6800 Mannheim 24 (DE)**
Erfinder : **Adolphi, Heinrich, Dr.**
**Kalmitweg 11**
**D-6703 Limburgerhof (DE)**

**EP 0 142 667 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft N-Benzoyl-N'-alkoxyphenylharnstoffe, Verfahren zu ihrer Herstellung und insektizide Mittel, die diese Verbindungen als Wirkstoffe enthalten.

Es ist bekannt, daß N-Benzoyl-N'-phenylharnstoffe insektizid wirksam sind (German Patent 2.123 236. French Patent 2,091 640, U.S. Patent 3 748 356).

Aus der DE-OS 3,046,672 sind insektizid wirksame N-Benzyl-N'-phenylharnstoffe bekannt, die sich durch einen N'-4-Cyclopropyloxyphenylrest als Substituenten auszeichnen. Diese Verbindungen sind jedoch relativ schlecht zugänglich, weil die zur Herstellung benötigten Cycloproproxyphenyl-isocyanate bzw. -aniline nicht leicht erhältlich sind.

Es wurde gefunden, daß N-Benzoyl, N'-(alkoxyphenyl) harnstoffe der Formel

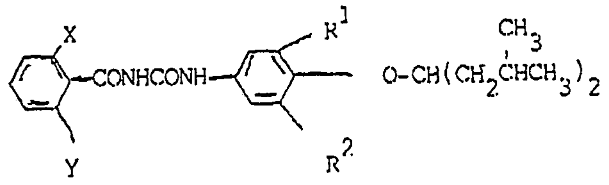

(I)

in der

entweder X und Y Fluor oder X Chlor und Y Wasserstoff bedeuten,

$R^1$ und $R^2$ (die gleich oder verschieden sein können) für H, F, Cl und Br

steht, insektizid sehr wirksam sind, sie sind bekannten Benzoylharnstoffen ähnlicher Struktur in ihrer Wirkung überlegen.

Man kann die N-Benzoyl-N'-phenylharnstoffe der Formel I unter anderem erhalten durch Umsetzung von Benzoylisocyanaten der Formel

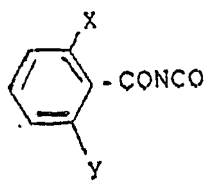

(II)

in der X und Y die obengenannten Bedeutungen haben, mit Alkoxyanilinen der Formel

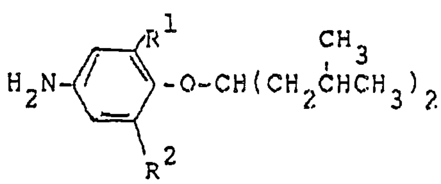

(III)

in der $R^1$ und $R^2$ die obengenannten Bedeutungen haben, vorzugsweise in Gegenwart eines inerten organischen Lösungsmittels bei einer ausreichenden Temperatur von im allgemeinen nicht mehr als 80 °C.

Für das Verfahren zur Herstellung der erfindungsgemäßen Verbindungen eignen sich eine Reihe von Lösungs- bzw. Verdünnungsmitteln. Als solche kommen z. B. in Betracht : Aliphatische und aromatische, gegebenenfalls chlorierte oder nitrierte Kohlenwasserstoffe, wie Benzol, Toluol, Xylole, Chlorbenzole, Benzin, Tetrachlorkohlenstoff, 1,2-Dichlorethan, Methylenchlorid, Chloroform, Nitromethan ; cyclische und acyclische Ether, wie Diethylether, Dibutylether, Tetrahydrofuran, Dioxan ; acyclische und cyclische Ketone, wie Aceton, Methylethylketon, Methylisopropylketon, Cyclohexanon ; Nitrile, wie Acetonitril, Benzonitril. Auch Gemische dieser Lösungsmittel können verwendet werden.

Zweckmäßig verwendet man ein Lösungsmittel (gemisch), in dem die Reaktionsprodukte unlöslich sind ; dies erleichtert deren Gewinnung.

Die Umsetzung verläuft exotherm und nimmt im Bereich zwischen 20 und 60 °C im allgemeinen eine ausreichende Geschwindigkeit an. Die Anwendung von überatmosphärischem Druck ist i. a. nicht erforderlich. Die Umsetzung kann diskontinuierlich oder kontinuierlich durchgeführt werden und verläuft praktisch quantitativ, wenn man die Reaktionsteilnehmer in etwa stöchiometrischem Verhältnis einsetzt. Ein Überschuß der einen oder anderen Komponente bringt keine wesentlichen Vorteile.

Die Harnstoffe der Formel I fallen gewöhnlich in reiner fester Form an — ggf. wird das Reaktionsgemisch teilweise eingeengt — und können falls nötig, durch Umkristallisieren gereinigt werden. Zu ihrer Charakterisierung dienen Elementaranalyse und Schmelzpunkt.

Zur Durchführung des erfindungsgemäßen Verfahrens wird zweckmäßigerweise das substituierte Alkoxyanilin zusammen mit dem Lösungs- oder Verdünnungsmittel vorgelegt, dann wird das Isocyanat zugegeben. Nach der Umsetzung, in der Regel nicht mehr als etwa 2 Stunden, wird das Produkt dann abgesaugt und unter vermindertem Druck getrocknet.

Die Benzoylisocyanate der Formel II und die Alkoxyaniline der Formel III und ihre Herstellung sind bekannt (J. Org. Chem. 28, 1805-1811 (1963) ; Weygand-Hilgetag Organisch-Chemische Experimentierkunst, 4. Auflage, 1970, S. 373, 576, J.A. Barth Verlag Leipzig) oder sie können nach bekannten Verfahren hergestellt werden.

Ein anderes Beispiel für die Herstellung von Harnstoffen der Formel I ist die Umsetzung von Amiden der Formel

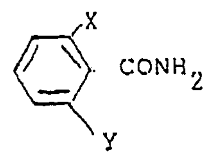

(IV)

mit Isocyanaten der Formel

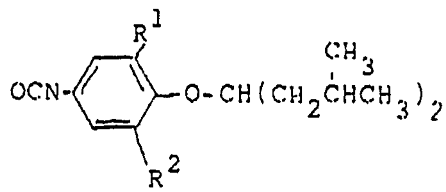

(V)

In den Formeln IV und V haben X, Y, $R^1$ und $R^2$ die obengenannten Bedeutungen.

Auch bei diesem Herstellverfahren werden die Reaktionspartner gewöhnlich in stöchiometrischen Mengen und vorzugsweise in Gegenwart eines Lösungsmittels umgesetzt. Eine ausreichende Reaktionstemperatur liegt i. a. unterhalb von 140 °C, vorzugsweise zwischen 60 und 100 °C. Gegebenenfalls kann ein Katalysator, wie Triethylamin oder eine Organozinnverbindung zugesetzt werden. Als Lösungsmittel kommen die gleichen Solventien in Betracht, die bei der Umsetzung der Verbindungen der Formel II mit den Verbindungen der Formel III verwendet werden können.

Herstellungsbeispiel 1

3,8 g (4-Amino, 2-fluorphenyl), (2,6-dimethylheptyl-4)-ether werden in 70 ml Toluol gelöst und mit 2,9 g 2-Chlorbenzoylisocyanat versetzt. Es wird über Nacht gerührt, eingeengt, abgesaugt und getrocknet.

3,8 g Harnstoff vom Fp.: 152-156 °C (Tabelle Nr. 2).

Durch entsprechende Anwandlung des vorstehenden Beispiels können die nachfolgend aufgeführten Verbindungen erhalten werden ; soweit sie hergestellt wurden, sind charakterisierende physikalische Stoffkonstanten (Schmelzpunkte) angegeben. Die nicht mit Stoffkonstanten versehenen Stoffe können auf die angegebene Weise erhalten werden und lassen aufgrund ihrer Strukturähnlichkeit eine den untersuchten Verbindungen vergleichbare biologische Wirksamkeit erwarten.

| Nr. | Y | X | $R^1$ | $R^2$ | Fp. °C |
|---|---|---|---|---|---|
| 1 | F | F | F | H | 117-124 |
| 2 | H | Cl | F | H | 152-156 |
| 3 | F | F | H | H | |
| 4 | H | Cl | H | H | 123-127 |
| 5 | F | F | Cl | Cl | |
| 6 | H | Cl | Cl | Cl | |
| 7 | F | F | H | Cl | 105-108 |
| 8 | H | Cl | H | Cl | 128-130 |

Die Benzoylalkoxyphenylharnstoffe der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden. Sie wirken vorzugsweise auf die Entwicklungsstadien ein, bei denen eine Häutung stattfindet (sog. Chitinsynthesehemmung).

Die erfindungsgemäßen Wirkstoffe werden auf die für Benzoylharnstoffe übliche Weise angewendet. Angaben zur Formulierung, Anwendungstechnik und Wirkungsweise und Angaben über geeignete Mischungspartner zur Erzielung synergistischer und anderer vorteilhafter Wirkungen können beispielsweise der US-PS 3,748,356 entnommen werden.

**Patentansprüche**

1. N-Benzoyl, N'-(alkoxyphenyl) harnstoffe der Formel

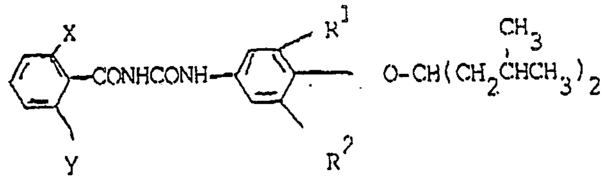

(I)

in der
entweder X und Y Fluor oder X Chlor und Y Wasserstoff bedeuten,
$R^1$ und $R^2$ (die gleich oder verschieden sein können) für H, F, Cl und Br
steht.

2. Insektizides und/oder akarizides Mittel, enthaltend einen N-Benzoylharnstoff der Formel I gemäß Anspruch I.

3. Insektizides und/oder akarizides Mittel, enthaltend inerte Zusatzstoffe und einen N-Benzoyl-N'-phenylharnstoff der Formel I gemäß Anspruch 1.

4. Verfahren zur Bekämpfung von Insekten und Akariden, dadurch gekennzeichnet, daß man eine insektizid wirksame Menge eines N-Benzoyl-N'-phenylharnstoffs der Formel I gemäß Anspruch 1 auf die Schädlinge und/oder deren Lebensraum einwirken läßt.

5. Verfahren zur Herstellung eines insektiziden und akariziden Mittels, dadurch gekennzeichnet, daß man mindestens einen N-Benzoyl-N'-phenylharnstoff der Formel I gemäß Anspruch 1 mit oberflächenaktiven Verbindungen und/oder Streckmitteln und/oder Synergisten mischt.

6. Verfahren zur Herstellung von N-Benzoyl-N'-phenylharnstoffen der Formel

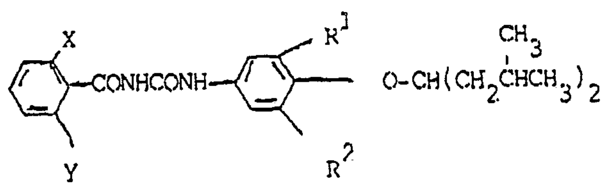

(I)

in der X, Y, $R^1$ und $R^2$ die obengenannte Bedeutung haben, dadurch gekennzeichnet, daß man ein Benzoylisocyanat der Formel

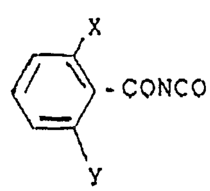

(II)

in der X und Y die obengenannten Bedeutungen haben, mit einem Alkoxyanilin der Formel

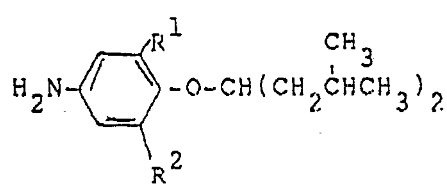

(III)

in der $R^1$, $R^2$ und $R^3$ die obengenannten Bedeutungen haben, in Gegenwart eines inerten organischen Lösungsmittels bei einer Temperatur im Bereich zwischen 0 und 80 °C umsetzt.

7. Verfahren zur Herstellung von N-Benzoyl-N'-phenylharnstoffen der Formel

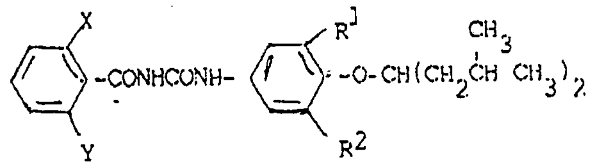

(I)

in der X, Y, $R^1$ und $R^2$ die obengenannten Bedeutungen haben, dadurch gekennzeichnet, daß man ein Amid der Formel

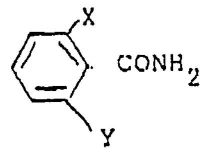

(IV)

in der X und Y die obengenannten Bedeutungen haben, mit einem Isocyanat der Formel

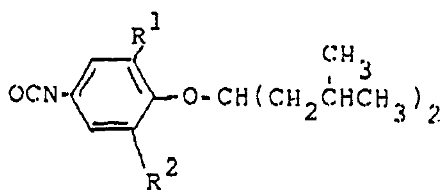

(V)

in der $R^1$ und $R^2$ die obengenannten Bedeutungen haben, gegebenenfalls in Gegenwart eines inerten organischen Lösungsmittels bei einer Temperatur zwischen 0 und 140 °C umsetzt.

**Claims**

1. An N-benzoyl-N'-alkoxyphenylurea of the formula

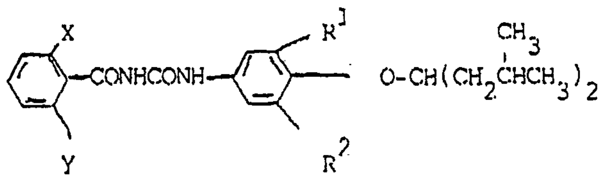

(I)

where
either X and Y are both fluorine or X is chlorine and Y is hydrogen and
$R^1$ and $R^2$ can be identical or different and are each H, F, Cl or Br.

2. An insecticidal and/or acaricidal agent, which contains an N-benzoylurea of the formula I as claimed in claim 1.

3. An insecticidal and/or acaricidal agent, which contains inert additives and an N-benzoyl-N'-phenylurea of the formula I as claimed in claim 1.

4. A method of controlling insects and acaridae, wherein an insecticidal amount of an N-benzoyl-N'-phenylurea of the formula I as claimed in claim 1 is allowed to act on the pests and/or their habitat.

5. A process for the preparation of an insecticidal and acaricidal agent, wherein one or more N-benzoyl-N'-phenylureas of the formula I as claimed in claim 1 are mixed with surfactants and/or extenders and/or synergistic agents.

6. A process for the preparation of an N-benzoyl-N'-phenylurea of the formula

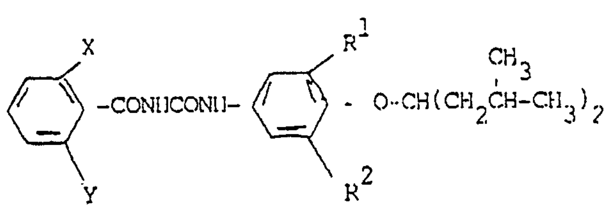

(I)

where X, Y, $R^1$ and $R^2$ have the above meanings, wherein a benzoyl isocyanate of the formula

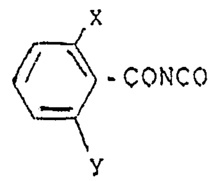

(II)

where X and Y have the above meanings, is reacted with an alkoxyaniline of the formula

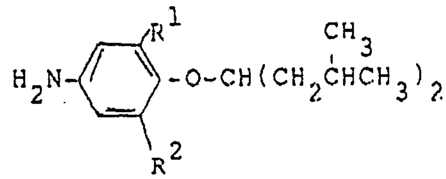

(III)

where $R^1$, $R^2$ and $R^3$ have the above meanings, in the presence of an inert organic solvent at from 0 to 80 °C.

7. A process for the preparation of an N-benzoyl-N'-phenylurea of the formula

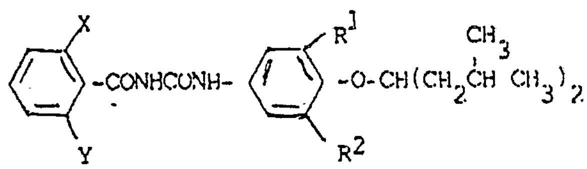

(I)

where X, Y, $R^1$ and $R^2$ have the above meanings, wherein an amide of the formula

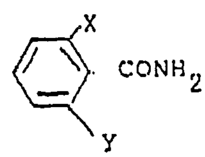

(IV)

where X and Y have the above meanings, is reacted with an isocyanate of the formula

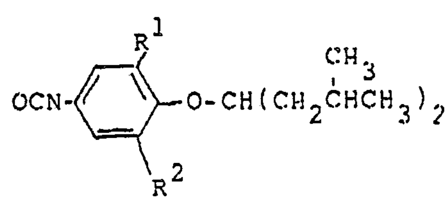

(V)

where $R^1$ and $R^2$ have the above meanings, in the presence or absence of an inert organic solvent at from 0 to 140 °C.

## Revendications

1. N-Benzoyl, N'-(alcoxyphényl)-urée de formule

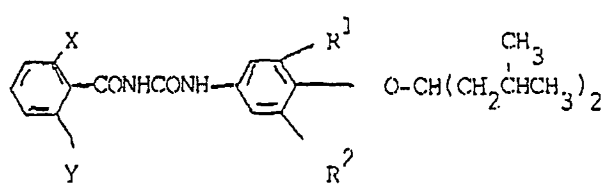

(I)

dans laquelle

soit X et Y représentent fluor, soit X représente chlore et Y hydrogène et

$R^1$ et $R^2$ (qui peuvent être identiques ou différents) sont mis pour H, F, Cl et Br.

2. Insecticides et/ou acaricides contenant une N-benzoyl-urée de formule I selon la revendication 1.

3. Insecticides et/ou acaricides contenant des additifs inertes et une N-benzoyl-N'-phénylurée de formule 1 selon la revendication 1.

4. Procédé pour lutter contre les insectes et acariens, caractérisé par le fait que l'on fait agir sur les parasites et/ou leur biotope une quantité efficace du point de vue insecticide d'une N-benzyl-N'-phénylurée de formule I selon la revendication 1.

5. Procédé de préparation d'un agent insecticide et acaricide, caractérisé par le fait que l'on mélange au moins une N-benzoyl-N'-phénylurée de formule 1 selon la revendication 1, avec des composés tensioactifs et/ou diluants et/ou synergistes.

6. Procédé de préparation de N-benzoyl-N'-phénylurée de formule

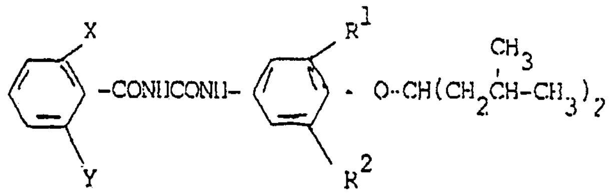

(I)

dans laquelle X, Y, $R^1$ et $R^2$ ont les significations sus-indiquées, caractérisé par le fait que l'on fait réagir, à une température comprise entre 0 et 80 °C, en présence d'un solvant organique inerte, un isocyanate de benzoyle de formule

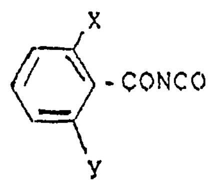

(II)

dans laquelle

X et Y ont les significations sus-indiquées, avec une alcoxyaniline de formule

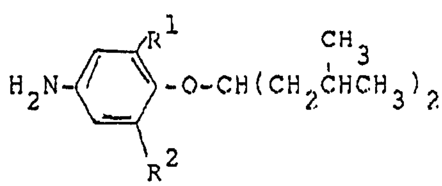

(III)

dans laquelle $R^1$, $R^2$ et $R^3$ ont les significations sus-indiquées.

7. Procédé de préparation de N-benzoyl-N'-phénylurée de formule

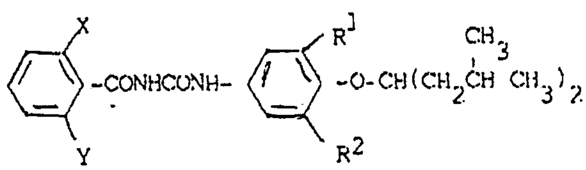

(I)

dans laquelle X, Y, $R^1$ et $R^2$ ont les significations sus-indiquées caractérisé par le fait que l'on fait réagir, à une température comprise entre 0 et 140 °C, éventuellement en présence d'un solvant organique inerte, un amide de formule

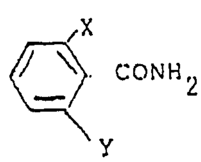

(IV)

dans laquelle X et Y ont les significations sus-indiquées avec un isocyanate de formule

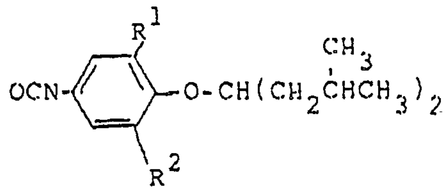

(V)

dans laquelle $R^1$ et $R^2$ ont les significations sus-indiquées.